# EUROPEAN PATENT APPLICATION

(11) **EP 1 707 195 A1**
(43) Date of publication of application: **04.10.2006**
(21) Application number: 05709250.4
(22) Date of filing: 21.01.2005
(51) Int. Cl.: A61K 31/203, A61P 9/10, A61P 3/10

(54) **RETINOIC ACID-CONTAINING REMEDY FOR DIABETES**

(30) Priority: 23.01.2004 JP 2004015236
(71) Applicant: Japan Science and Technology Agency, Kawaguchi-shi, Saitama 332-0012 (JP)
(72) Inventor: YAMAGUCHI, Yoko;, Kanagawa 2580022 (JP); IGARASHI, Rie;, Kanagawa 2140036 (JP)
(74) Representative: Walcher, Armin
(86) International application number: PCT/JP2005/000743
(87) International publication number: WO 2005/070413

(57) **Abstract**

The present invention aims to provide a novel agent for treating and/or preventing diabetes which agent can not only control a blood sugar level but also fundamentally treat a patient with type I diabetes suffering from destruction of β cells and a patient with type II diabetes suffering from dysfunction in insulin secretion. The present invention provides an agent for treating and/or preventing diabetes, the agent containing retinoic acid as an active ingredient. Retinoic acid incorporated as an active ingredient may be all-trans retinoic acid, an isomer thereof, a derivative thereof, a salt thereof or a prodrug thereof. Retinoic acid may be incorporated singly. Alternatively, composite particles of retinoic acid and an appropriate inorganic or organic substance are prepared, and the retinoic acid composite particles may be incorporated.

## Description

### Technical Field

The present invention relates to an agent for preventing and/or treating diabetes or its related diseases, the agent containing retinoic acid as an active ingredient. More specifically, it relates to an antidiabetic agent which not only controls a blood sugar level but also directly acts on β cells of pancreas.

### Background Art

It has been said that there are now approximately 6,000,000 diabetic patients in our country, and there is also a statistic data that one of ten persons who are more than 40 years old in particular suffer from diabetes. The diabetes is defined as "a group of metabolic diseases having a main symptom of chronic hyperglycemia due to insufficient function of insulin" (Japan Diabetes Society, Committee Report on Classification and Diagnostic Criteria of Diabetes, 1999). It is mainly classified into type I diabetes caused for lack of insulin by destruction of P cells of pancreas secreting insulin for some cause and type II diabetes caused by both of a state of decrease in insulin secretion from pancreas and a state of an insufficient effect of insulin (a state of insulin resistance). When a condition of hyperglycemia continues, it may induce severe diseases such as retinopathy, nephropathy and neuropathy which are said to be three great complications of diabetes, stroke and myocardial infarction.

In recent years, an onset mechanism of hyperglycemia is being clarified. In addition to ordinary diabetic therapy mainly using an oral agent such as a sulfonylurea (SU) agent and an insulin pharmaceutical preparation, a biguanide (BG) agent which inhibits glucose regeneration or release in liver or accelerates use or storage of glucose in skeletal muscles or fat cells, an α-glucosidase inhibitor which decreases glucose absorption in intestines, an insulin activity enhancer (an insulin resistance improving agent) which directly enhances the insulin activity without promoting insulin secretion, and the like have been used on the basis of a new functional mechanism (non-Patent Document 1). As an agent which directly acts on β cells of pancreas, NN-623, AY 4166 and the like which are novel insulin secretion accelerators other than the SU agent have been known (non-Patent Document 1).

These oral agents are effective for patients who keep insulin secretion, but not effective for patients with type I diabetes who are deficient in insulin secretion, and insulin injection for suppressing a blood sugar level is indispensable to such patients. The insulin injection is sometimes required when even patients with type II diabetes cannot sufficiently control the blood sugar level by therapy of diet and movement and with oral agents, serious infectious diseases such as serious pneumonia concurrently occur, patients are operating, serious renal diseases or serious liver diseases concurrently occur or patients are in diabetic coma.
non-Patent Document 1: Ohta Masao, Journal of Nippon Medical School, 1999, vol. 66, No. 3, pp. 39-42

### Disclosure of the Invention

### Problems that the Invention is to Solve

As stated above, the ordinary antidiabetic agents mainly aim to control the blood sugar level, and the patients with type I diabetes suffering from destruction of β cells and the patients with type II diabetes suffering from dysfunction in insulin secretion have at present no choice but to rely on intricate insulin injection. Accordingly, medical agents which can fundamentally treat diabetes by directly acting on pancreas which undergoes destruction or dysfunction have been in demand.

### Means for Solving the Problems

The present inventors have so far independently studied retinoic acid, and have found for the first time that retinoic acid, when administered in vivo, recovers the function of pancreas. This finding has led to the completion of the present invention.

Accordingly, the present invention provides an agent for treating and/or preventing diabetes, the agent containing retinoic acid as an active ingredient, whereby the foregoing problems can be solved.

### Effect of the Invention

The agent for treating and/or preventing diabetes according to the present invention is considered to recover the function of pancreas (β cells) by directly acting on pancreas. Therefore, the agent of the present invention is effective not only for controlling the blood sugar level of the patients with insulin-resistant type II diabetes but also for the patients with type I diabetes suffering from destruction of β cells.

Further, since the agent can keep the blood sugar level in an appropriate range by acting on pancreas containing β cells to improve the function of insulin secretion, it is also effective for preventing and treating various complications accompanied by hyperglycemia and the like.

### Brief Description of the Drawings

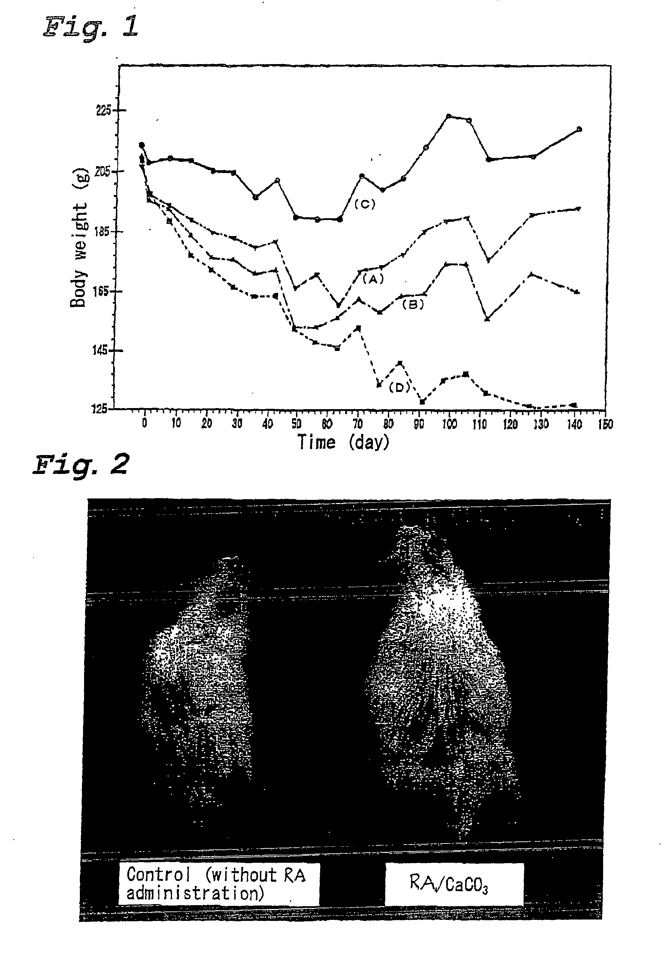

[Fig. 1] It is a graph showing a change in body weight of diabetic rats with or without administration of retinoic acid. (A) rat with administration of retinoic acid (6 mg); (B) rat with administration of retinoic acid-inorganic substance (CaCo₃) composite particles (6 mg); (C) rat with administration of retinoic acid-organic substance (PLGA) composite particles (6 mg); (D) control rat (without administration of retinoic acid).
[Fig. 2] It is photos showing appearances on day 100 of a diabetic rat with administration of retinoic acid-inorganic substance composite particles (RA/CaCo₃) (6 mg) and a control diabetic rat (without RA administration).

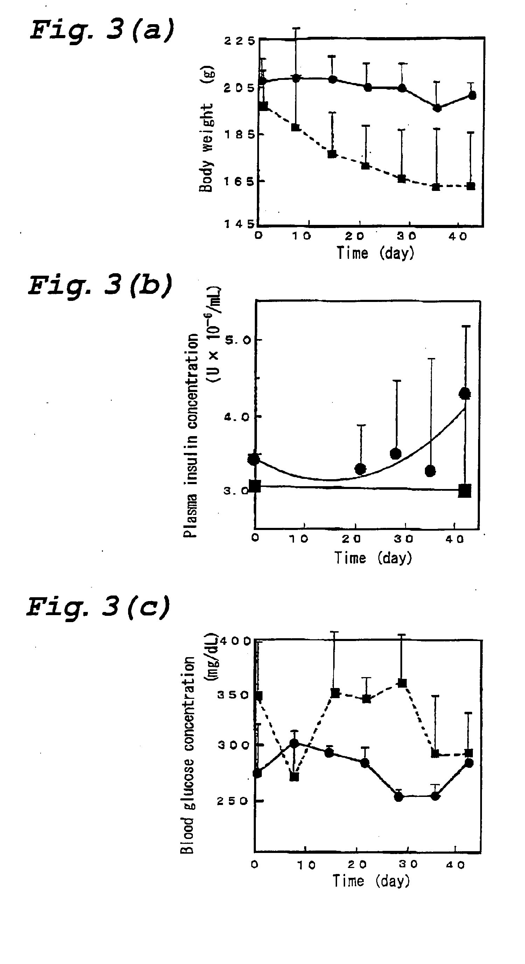

[Fig. 3] It is a graph showing (a) a change in body weight, (b) a change in plasma insulin concentration, and (c) a change in blood glucose concentration, of diabetic rats with or without administration of retinoic acid. •: rat with administration of retinoic acid-organic substance (PLGA) composite particles (6 mg); ■: control rat (without administration of retinoic acid).

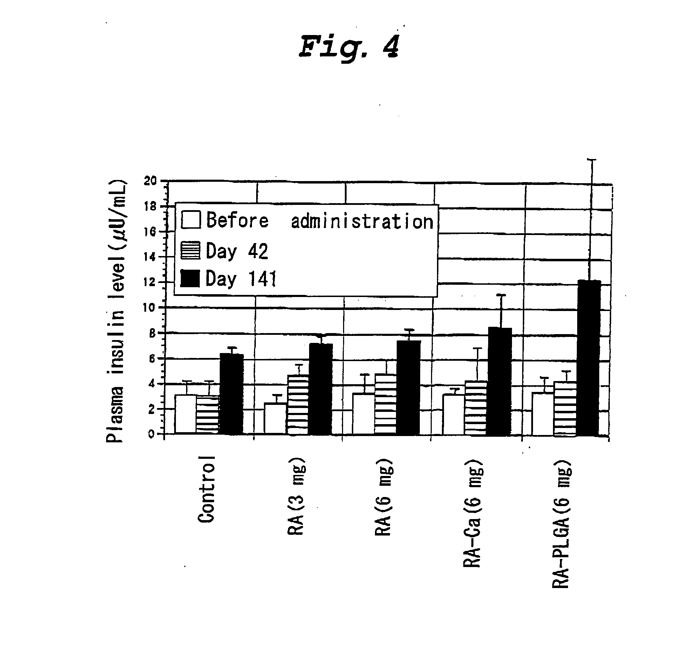

[Fig. 4] It is a graph showing a change in plasma insulin concentration of diabetic rats with or without administration of retinoic acid. RA: rat with administration of retinoic acid; Ra-Ca: rat with administration of retinoic acid-inorganic substance (CaCo₃) composite particles; RA-PLGA: rat with administration of retinoic acid-organic substance (PLGA) composite particles; control: rat without administration of retinoic acid. The parenthesized value indicates a dose.

### Best Mode for Carrying Out the Invention

The agent of the present invention is characterized by containing retinoic acid as an active ingredient. Retinoic acid is a liposoluble small molecule derived from vitamin A, and all-trans retinoic acid and 9-cis-retinoic acid are known as a main isomer. In the agent of the present invention, all-trans retinoic acid represented by the following formula (I) is preferably used.

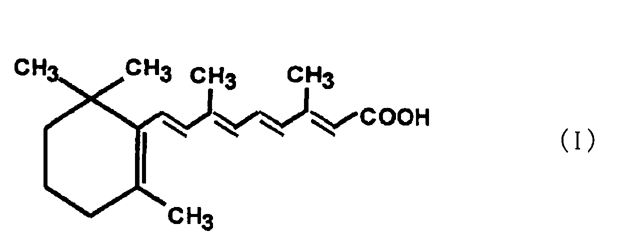

Retinoic acid has a function of inducing differentiation of undifferentiated cells, and it has been suggested that retinoic acid exhibits a morphogen-like function in morphogenesis. On the basis of this function of inducing differentiation, it has been clinically used as a therapeutic agent of acute promyelocytic leukemia (APL). Moreover in the latest investigation of regenerative medicine, a method in which an upper labial part of a blastopore in an initial gastrula of Xenopus is treated with retinoic acid and then cultured to induce differentiation to pancreas in vitro has been proposed (gazette of JP-A-2001-299335).

That is, retinoic acid regenerates in vitro pancreas during embryogenesis by controlling differentiation of undifferentiated cells of higher organisms. However, what has been studied so far is directed to totipotent embryonal stem cells having a possibility of differentiation to all tissues (organs). No knowledge has been obtained as to what function retinoic acid exhibits in vivo to somatic stem cells derived from each organ. The present invention is based on a new knowledge that retinoic acid acts on pancreas in vivo so as to be able to recover the insulin secreting function of β cells and improve a blood sugar level controlling function.

Retinoic acid used as an active ingredient of the agent of the present invention is preferably all-trans retinoic acid represented by the above formula (I). Its isomers, derivatives such as esters, salts or prodrugs are also available.

In the agent of the present invention, retinoic acid may be incorporated as such. It is also advisable that composite particles of an appropriate inorganic or organic substance and retinoic acid are prepared and the very retinoic acid composite particles are incorporated as an active ingredient.

Examples of the retinoic acid composite particles include particles of retinoic acid surface-modified with an inorganic substance such as a carbonate or a phosphate of calcium or zinc or an organic substance such as a biodegradable polymer, solid particles integrated with this substance, and the like. These retinoic acid composite particles can be in the form of coating the whole or a part of the surface of retinoic acid with the inorganic or organic substance or in the form of dispersing retinoic acid in solid particles made of the inorganic or organic substance. However, these forms are not critical. The use of such composite particles lessens irritation of retinoic acid itself, decreases a risk of tumor formation even by subcutaneous injection and secures controlled release of retinoic acid as an active substance which is included in the particles.

The retinoic acid-inorganic substance composite particles can be prepared by using, for example, an amphipathic property of retinoic acid. Specifically, for example, calcium carbonate is selected as an inorganic substance. First, retinoic acid is dispersed in an alkaline aqueous medium along with a small amount of a polar organic solvent to form spherical micelles whose surfaces are covered with a minus charge. At this time, a nonionic surfactant is added to form mixed micelles of retinoic acid and the nonionic surfactant, whereby agglomeration and precipitation of micelles can be prevented. Further, calcium chloride is added to adsorb calcium ions (Ca²⁺) on minus-charged surfaces of micelles to form spherical or oval micelles whose surfaces are coated with calcium ions. In addition, sodium carbonate is added to adsorb (bind) inorganic acid ions such as carbonate ions (CO₃²⁻) on calcium ions of the micelle surfaces to neutralize the micelle surface charge. Consequently, a film of calcium carbonate is formed on the surfaces of the retinoic acid particles to obtain retinoic acid composite particles surface-modified with the inorganic substance.

Examples of the polar solvent used in dispersing retinoic acid include ethanol, methanol, acetone, ethyl acetate, dimethyl sulfoxide and the like. In the percutaneous administration, ethanol or methanol which is less irritant is preferable.

As the inorganic substance, inorganic acid salts such as a carbonate and a phosphate, of polyvalent metals, especially divalent metals, such as calcium, magnesium and zinc are used. Polyvalent metal inorganic acid salts having a biocompatibility are preferable.

Polyvalent metals are added in the form of a chloride, an acetate, a halide and the like, for example, calcium chloride, magnesium chloride and zinc acetate. Inorganic acids are added in the form of alkali metal salts, for example, sodium carbonate and sodium phosphate.

Examples of the nonionic surfactant used in the foregoing preparation method include polyoxyethylene (20) sorbitan monooleate, polyoxyethylene (20) sorbitan monolaurate, polyoxyethylene (20) sorbitan monostearate, polyoxyethylene (20) sorbitan monopalmitate, polyoxyethylene (20) sorbitan trioleate, polyoxyethylene (8) octylphenyl ether, polyoxyethylene (20) cholesterol ester, polyoxyethylene (30) cholesterol ester, polyoxyethylene hardened castor oil and the like. As a commercially available product, Tween 80 and the like can be mentioned.

When the weight ratio of the polyvalent metal (chloride or the like) and the inorganic acid (sodium salt or the like) to be added is from 1:0.05 to 1:0.33, preferably 1: approximately 0.2, a transparent aqueous suspension of retinoic acid-inorganic substance composite particles having a particle diameter of from approximately 5 to 1,000 nm can be obtained.

Moreover, the present inventors have found that during the formation of the film of the polyvalent metal inorganic salt on the surface of the micelle of retinoic acid, the molar ratio of the polyvalent metal salt such as calcium chloride and the alkali metal salt of the inorganic acid such as carbonic acid or phosphoric acid which are added is adjusted to the range of from approximately 1:0 to 1:1.0, whereby composite particles having an average particle diameter of from approximately 5 to 300 nm (nanoparticles) can be prepared. The composite particles having such a small particle diameter in the nanometer order are especially preferable when retinoic acid is administered subcutaneously or intravenously, or percutaneously absorbed by skin administration (coating administration or the like).

The polyvalent metal inorganic acid salt such as calcium carbonate which is formed on the micelle surface in the foregoing method has an amorphous with a so-called vitreous form or semi-stable phase vaterite structure. Accordingly, a solubility in water of the film is high, and a biodegradability is improved to allow easy decomposition. Consequently, it has been confirmed that when the resulting retinoic acid-calcium carbonate composite particles are administered in living body, the calcium carbonate film layer on the micelle surface is easily decomposed to release retinoic acid included therein, with the result that the pharmaceutical effect can be exhibited in a controlled manner.

In practice, when the aqueous suspension of the retinoic acid-calcium carbonate composite particles is subcutaneously injected in a rat or the composite particles are coated on the surface of skin along with a vaseline base, a quite excellent absorbability has been exhibited in comparison to the case of using retinoic acid alone without causing tumor formation on the administered site.

Meanwhile, the composite particles of the organic substance and retinoic acid can be prepared by a general particle formation method (drying-in-liquid method) using a biodegradable polymer such as a lactic acid/glycolic acid copolymer (PLGA) or polylactic acid (PGA). Specifically, the particles are prepared by dispersing retinoic acid in a halogenated hydrocarbon solution of a biodegradable polymer polyvalent metal salt, dispersing the resulting dispersion in a polyvinyl alcohol aqueous solution or the like, evaporating the halogenated hydrocarbon to form particles and washing the resulting particles with distilled water or the like.

As the lactic acid/glycolic acid copolymer used as the biodegradable polymer, a D-isomer, an L-isomer or a mixture of these is available, and a racemic compound is preferably used. The copolymerization ratio of the lactic acid/glycolic acid copolymer (lactic acid/glycolic acid; mol%) is preferably from approximately 95/5 to 45/55. It is more preferably from 90/10 to 45/55. Its weight average molecular weight is preferably from approximately 3,000 to 20,000, more preferably from 3,000 to 15,000. A degree of dispersion of the lactic acid/glycolic acid copolymer (weight average molecular weight/number average molecular weight) is from 1.2 to 4.0, preferably from approximately 1.2 to 2.5. These copolymers are synthesized by a method known per se. Of these, a copolymer produced by a non-catalytic dehydrative condensation method is preferable.

In the present invention, two or more lactic acid/glycolic acid copolymers different in copolymerization ratio and weight average molecular weight may be used by being mixed at an optional ratio. For example, a mixture of a copolymer having a copolymerization ratio of 50/50 and a weight average molecular weight of approximately 6,000 and a copolymer having a copolymerization ratio of 50/50 and a weight average molecular weight of approximately 3,000 is used. An appropriate weight ratio in the mixing is from 75/25 to 25/75. The weight average molecular weight and the number average molecular weight in the present specification mean values measured by gel permeation chromatography using polystyrene as a standard substance.

In the foregoing preparation method, the polyvalent metal salt or oxide used along with the biodegradable polymer is not particularly limited so long as it is a metal salt or oxide that does not have an adverse effect on living body. Salts of alkaline earth metals (calcium, magnesium and the like), zinc, iron, copper, tin, aluminum and the like and inorganic or organic acids, or oxides of these metals are used. Metals are preferably alkaline earth metals, zinc, iron and aluminum, and zinc and calcium are used especially preferably. Examples of inorganic acids include hydrochloric acid, sulfuric acid, nitric acid, thiocyanic acid and the like. As the organic acids, aliphatic carboxylic acid and aromatic carboxylic acids are used.

As the halogenated hydrocarbon which is a solvent, a chlorinated saturated hydrocarbon having 1 or 2 carbon atoms and a fluorinated saturated hydrocarbon having 1 or 2 carbon atoms are preferably used. Specific examples thereof can include chloroform, methylene chloride, 1,2-dichloroethane, 1,1-dichloroethane, monofluoromethane, difluoromethane and the like. The solution contains a biodegradable polymer and a polyvalent metal salt or oxide in amounts of, preferably from 10 to 90% by weight and from 0.01 to 3% by weight, more preferably from 30 to 80% by weight and from 0.01 to 2% by weight respectively based on the total amount of the biodegradable polymer, the polyvalent metal salt or oxide and the halogenated hydrocarbon.

In the foregoing drying-in-liquid method, another organic solvent miscible with water, such as an aliphatic organic compound having from 1 to 3 hydroxyl groups, may be used instead of polyvinyl alcohol. Examples of such a compound include glycerin, polyethylene glycol, propylene glycol, ethyl alcohol, isopropyl alcohol, benzyl alcohol and the like.

The agent of the present invention is usually provided in the form of a composition obtained by mixing retinoic acid or retinoic acid composite particles of retinoic acid and an inorganic or organic substance with pharmacologically acceptable carriers. As the pharmacologically acceptable carriers here referred to, various organic or inorganic carrier substances which are ordinarily employed in the field of pharmaceutical preparations may be used.

When liquid pharmaceutical preparations such as an injection solution are prepared, retinoic acid or the retinoic acid composite particles are dispersed or dissolved in a medium such as injection water, a physiological saline solution, a Ringer solution, alcohol, propylene glycol, polyethylene glycol, sesame oil, corn oil, olive oil or cottonseed oil. Especially, an aqueous suspension obtained by dispersing retinoic acid in an aqueous medium is preferable. Liquid pharmaceutical preparations may contain, besides the foregoing medium, additional components such as a solubilizer, a suspending agent, an isotonizing agent, a buffer agent, an antiseptic and an antioxidant.

The agent of the present invention may be provided in the form of a pharmaceutical preparation obtained by freeze-drying the foregoing liquid pharmaceutical preparation or a solid pharmaceutical preparation obtained by solidifying the active ingredient. It may be formed into an injection solution by being dispersed or dissolved in an appropriate solvent when used. In case of the solid pharmaceutical preparation, additional components such as an excipient, a binder or a disintegrant may be incorporated.

The dosage form of the agent of the present invention is intended for the liquid pharmaceutical preparation such as the injection solution. However, parenteral agents such as an external preparation (an ointment or the like), a suppository, a pellet, a transnasal agent and an inhalant or oral agents such as a capsule are also available.

The thus-prepared agent of the present invention is effective for preventing and/or treating diabetes (including type I diabetes and type II diabetes) and also for preventing and/or treating various diseases accompanied by hyperglycemia.

The dose of the agent of the present invention varies with the administration patients, the administration route, the conditions of diseases and the like. For example, when the agent is orally administered to an adult diabetic patient, a dose of retinoic acid as an active ingredient in one administration is from approximately 0.01 to 100 mg/kg, preferably from 0.05 to 30 mg, more preferably from 0.1 to 10 mg per kilogram of body weight, and it is preferable to administer the agent at this dose once to three times a day.

The agent of the present invention is characterized by directly acting on P cells of pancreas to improve diabetes and return the blood sugar level to a normal level, and a risk of onset of the hypoglycemic symptom caused by the ordinary blood sugar controlling agent is considered to be low. Accordingly, it may be used in combination with an ordinary agent intended for controlling a blood sugar level, such as an insulin pharmaceutical preparation, an insulin resistance improving agent, an α-glucosidase inhibitor, a biguanide agent or an insulin secretion accelerator (SU agent).

### Example 1

Preparation of a retinoic acid pharmaceutical preparation

As retinoic acid, commercially available retinoic acid (solid particles) was used as such.

### (1) Preparation of retinoic acid-inorganic substance composite particles

13.6 mg of retinoic acid was dissolved in 900 µL of ethanol (or methanol), and 100 µL of 0.5 N NaOH aqueous solution was added to this solution. At this time, the pH was from 7 to 7.5. As a mother liquor, 100 µL of this solution was collected, and added to 100 µL of distilled water containing Tween 80 (trade name), and the mixture was stirred well. After approximately 30 minutes, 5 M calcium chloride-containing aqueous solution was added, and the mixture was stirred. After 30 minutes, 1 M sodium carbonate-containing aqueous solution was added, and the mixture was further stirred. After the stirring continued for 24 hours, the resulting solution was freeze-dried overnight to prepare desired retinoic acid-calcium carbonate composite particles.

### (2) Preparation of retinoic acid-organic substance composite particles

PLGA (7.16 g), ZnO (40 mg) and retinoic acid (800 mg) were charged into dichloromethane, and uniformly dispersed with a vortex mixer. Subsequently, the resulting solution was poured via a vial into distilled water (1,600 mL) in which 16 g of polyvinyl alcohol (molecular weight 45,000) and 11.2 g of zinc acetate had been dissolved, and the mixture was stirred for approximately 3 hours to evaporate dichloromethane. The particles formed by evaporation of dichloromethane were separated from distilled water through a centrifugal separator. The particles separated as a precipitate were washed with distilled water several times, and then subjected to wet filtration with a sieve of 125 µm to make uniform the particle size. Finally, the particles were dried with a freeze-drier for approximately 24 hours.

The above-prepared retinoic acid composite particles of retinoic acid and the inorganic and organic substances were dispersed in a physiological saline solution at a predetermined concentration (from 3 to 12 mg/mL) to prepare an injection solution.

### Example 2

The injection solution (containing 6 mg of retinoic acid) prepared in Example 1 was subcutaneously injected in the back of each diabetic rat at a dose of 1 mL via a syringe fitted with an injection needle of from 18 to 21 gauge. When a needle of 18 gauge was used, the site was encapsulated in a surgical Aron Alpha (trade name) after the injection to prevent liquid leakage.

Subsequently, the change in body weight of each rat was observed, and the results are shown in Fig. 1. As is seen from Fig. 1, a control rat (D) without administration of retinoic acid was gradually decreased in body weight. Meanwhile, in retinoic acid administration rats (A) to (C), the decrease in body weight was initially observed, but a tendency of increasing body weight was then observed. Especially, in the rat (C) to which the pharmaceutical preparation of the retinoic acid-organic substance composite particles was administered, body weight was decreased for a short period of time, but thereafter increased.

Photos showing appearances of the retinoic acid (inorganic substance composite particles) administration rat and the control rat (without administration of retinoic acid) on day 100 after starting the measurement are shown in Fig. 2. It has been found that the control rat is thin because of the decrease in body weight, whereas the retinoic acid administration rat is recovered by suppressing the decrease in body weight.

The same tendency was observed when the dose of retinoic acid was changed to 3 mg and 12 mg.

### Example 3

The change in body weight until approximately day 40 of the diabetic rat with the administration of retinoic acid-organic substance (PLGA) composite particles (6 mg) and the control diabetic rat (without administration of retinoic acid) are shown in Fig. 3(a), and the change in plasma insulin concentration and the change in blood glucose concentration of the rats at the same stage are shown in Figs. 3 (b) and 3 (c) . In the drawings, • indicates the rat with the administration of retinoic acid-organic substance (PLGA) composite particles (6 mg), and ■ indicates the control rat (without administration of retinoic acid).

In the control rat without administration of retinoic acid, there was observed a tendency that plasma insulin concentration was unchanged or gradually decreased, whereas in the rat with the administration of retinoic acid-organic substance composite particles (RA/PLGA), plasma insulin concentration was lowest approximately on day 15, and then increased (Fig. 3(b)).

Further, in Fig. 3(c) showing the change in blood glucose concentration, the data is quite irregular because the measurement is conducted on condition that the rat is not fasted. Meanwhile, upon comparing Fig. 3(c) with Figs. 3 (a) and 3(b), there is observed a tendency that blood glucose concentration (blood sugar level) is increased or decreased according to the change in plasma insulin concentration. It can be said that these results prove that the change in body weight shown in Fig. 1 is due to the progression of diabetes (control rat) and recovery from diabetes (administration rat).

Retinoic acid does not have a function of directly stimulating insulin secretion. Accordingly, it can be considered that a function of β cells of pancreas is recovered by administering retinoic acid to improve an insulin secretion activity, whereby blood glucose concentration is suppressed to lessen the diabetic symptoms.

### Example 4

It is Fig. 4 that shows results of comparing changes in plasma insulin concentration of rats under various conditions before and after retinoic acid administration. Upon comparing the values before administration and on day 42 after administration, it has been found that insulin concentration remains unchanged in the control rat without administration of retinoic acid, whereas the insulin level is increased in the rat with administration of retinoic acid under any conditions in comparison to the insulin level before administration. Also on day 141 after administration, it has been found that the insulin level of the administration rat tends to be high in comparison to the control rat.

## Claims

1. An agent for treating or preventing diabetes, the agent containing retinoic acid as an active ingredient.

2. The agent as claimed in claim 1, wherein the retinoic acid is all-trans retinoic acid represented by the following formula (I).

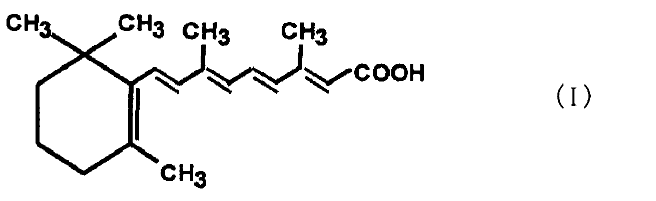

3. The agent as claimed in claim 1, which is in the form of an aqueous suspension of retinoic acid.

4. The agent as claimed in claim 1, wherein retinoic acid is in the form of composite particles of retinoic acid and an inorganic substance or an organic substance.

5. The agent as claimed in claim 4, wherein the inorganic substance is a polyvalent metal inorganic acid salt.

6. The agent as claimed in claim 4, wherein the organic substance is a biodegradable polymer.

7. The agent as claimed in claim 4, wherein the composite particles are nanoparticles having an average particle diameter of from 5 to 300 nm.

8. An agent for activating β cells of pancreas, the agent containing retinoic acid as an active ingredient.
